# FASCICULE DE BREVET EUROPEEN

(11) **EP 3 853 380 B1**
(45) Date de publication et mention de la délivrance du brevet: **07.01.2026**
(21) Numéro de dépôt: 19766278.6
(22) Date de dépôt: 17.09.2019
(51) Int. Cl.: C12Q 1/6832, C12Q 1/6841

(54) **PROCEDE A HAUT DEBIT POUR DETECTER DES ABERRATIONS CHROMOSOMIQUES ET/OU DES ABERRATIONS DE TELOMERES**
HOCHDURCHSATZVERFAHREN ZUR ERFASSUNG CHROMOSOMISCHER ABERRATIONEN UND / ODER TELOMERISCHER ABERRATIONEN
HIGH THROUGH PUT PROCESS FOR DETECTING CHROMOSOMIC ABERRATIONS AND/OR TELOMERIC ABERRATIONS

(30) Priorité: 18.09.2018 FR 1858427
(43) Date de publication de la demande: 28.07.2021
(73) Titulaire: Cell Environment, 94500 Champigny-sur-Marne (FR)
(72) Inventeur: M'KACHER, Radhia, 94500 CHAMPIGNY SUR MARNE (FR)
(74) Mandataire: IPAZ
(86) Numéro de dépôt international: PCT/EP2019/074870
(87) Numéro de publication internationale: WO 2020/058268

(56) Documents cités:
- WO-A2-97/14026
- CN-A- 108 004 299
- JP-A- 2006 242 618
- IOANNA KARACHRISTOU ET AL: "Triage biodosimetry using centromeric/telomeric PNA probes and Giemsa staining to score dicentrics or excess fragments in non-stimulated lymphocyte prematurely condensed chromosomes", MUTATION RESEARCH. GENETIC TOXICOLOGY AND ENVIRONMENTAL MUTAGENESIS, vol. 793, 6 November 2015 (2015-11-06), NL, pages 107 - 114, XP055568762, ISSN: 1383-5718, DOI: 10.1016/j.mrgentox.2015.06.013
- DEMETRE ZAFIROPOULOS ET AL: "Biological dosimetry of ionizing radiation: Evaluation of the dose with cytogenetic methodologies by the construction of calibration curves", INTERNATIONAL JOURNAL OF MODERN PHYSICS: CONFERENCE SERIES, vol. 44, 1 September 2016 (2016-09-01), pages 1660239, XP055568765, DOI: 10.1142/S2010194516602398
- RADHIA M'KACHER ET AL: "Detection and Automated Scoring of Dicentric Chromosomes in Nonstimulated Lymphocyte Prematurely Condensed Chromosomes After Telomere and Centromere Staining", INTERNATIONAL JOURNAL OF RADIATION: ONCOLOGY BIOLOGY PHYSICS., vol. 91, no. 3, 1 March 2015 (2015-03-01), USA, pages 640 - 649, XP055646769, ISSN: 0360-3016, DOI: 10.1016/j.ijrobp.2014.10.048

## Description

La présente demande concerne un procédé à haut débit pour détecter des aberrations chromosomiques et/ou des aberrations de télomères.

Au cours de la dernière décennie, de nombreux travaux de recherches ont démontré le rôle majeur des télomères dans l'intégrité et la stabilité du génome. A chaque division cellulaire, les télomères constituant l'extrémité de chaque chromosome se raccourcissent. Quand les télomères deviennent trop courts, et avant que les gènes ne soient affectés ou que les chromosomes fusionnent entre eux, la cellule cesse de se diviser et entre en sénescence. Les télomères sont donc considérés comme une horloge biologique qui gouverne le vieillissement des cellules. Il est désormais bien documenté que la longueur des télomères est un biomarqueur important dans les maladies liées au vieillissement comme les cancers et les maladies cardiovasculaires athérosclérotiques. [Hubert J et al, Circ Res.16;122(4):616-623 2018 ; Staerk L et al, J Am Heart Assoc. 2017 Nov 14;6(11)].

La détermination de la longueur des télomères et des aberrations chromosomiques n'a pas seulement des implications pratiques pour les cliniciens dans la prise en charge thérapeutique des patients mais ouvre également de nouveaux champs de recherche et des applications en recherche clinique, et en particulier au cours d'essais thérapeutiques.

L'étude de la longueur des télomères est donc cruciale pour la compréhension des mécanismes cellulaires, l'évaluation et la prise en charge des patients. A ce jour, plusieurs techniques d'analyse existent.

L'analyse des fragments de restriction terminaux (TRF ou Terminal Restriction Fragmentation) est la première méthode développée pour déterminer la longueur des télomères. Elle est souvent considérée comme la méthode de référence. Cette méthode permet d'estimer le nombre moyen de répétitions terminales portées par l'ensemble des chromosomes. La procédure consiste en la digestion de l'ADN génomique par une ou plusieurs enzymes de restriction. Les fragments sont ensuite séparés par électrophorèse en fonction de leur taille. La taille des fragments terminaux est estimée par comparaison avec des fragments de longueur connue. [Allshire, Dempster & Hastie, Nucleic Acids Res. 1989 Jun 26;17(12):4611-27]. Cette méthode présente l'inconvénient d'être longue et fastidieuse et nécessite de grandes quantités d'ADN (>1µg). De plus, elle conduit à l'obtention d'une valeur moyenne de longueur et non à la mise en évidence individuelle du raccourcissement ou de l'absence de télomère.

La technique de PCR quantitatif, par exemple MMqPCR, aTL qPCR, a été développée afin de contourner l'obstacle de l'utilisation de grandes quantités d'ADN. Cependant, l'un des principaux inconvénients de cette méthode est que l'on observe de grandes variations de mesures entre différents aliquotes. De plus, comme pour la méthode TRF, les valeurs mesurées correspondent à une moyenne.

La méthode Q-FISH (L'hybridation in-situ de fluorescence, éventuellement combinée avec la cytométrie en flux, permet la visualisation des télomères par hybridation en utilisant une sonde de fluorescence sur des métaphases. [Lansdorp et al. Hum Mol Genet. 1996 May;5(5):685-91.]. L'avantage de cette technique est qu'elle permet d'estimer la taille individuellement de chacun des 184 télomères humains et n'est pas limitée à une moyenne ou aux petits télomères. En revanche la méthode de quantification sur métaphases était très laborieuse à mettre en pratique et les longueurs sont exprimées en unité de fluorescence relative.

En 2007, l'équipe de Maria Blasco [Canela et al. Proc NatI Acad Sci U S A. 2007 Mar 27;104(13):5300-5] a décrit une nouvelle technique basée sur le Q-FISH mais sur des noyaux interphasiques en utilisant l'objectif de grossissement 63X dans la quantification globale des télomères et une plaque de 96 puits pour les cultures des cellules et les hybridations. Cependant, cette technique ne permet pas la quantification des télomères de chaque chromosome ni la détection des aberrations télomériques. D'ailleurs, la technique de l'équipe de Maria Blasco reste longue et laborieuse, car elle ne permet que l'analyse d'environ 1000 cellules en 2 heures.

Ioanna Karachriston et al (Mutation Research 793(2015) 107-114) décrivent l'utilisation de la technique de « premature chromosome condensation (PCC) » par la fusion des lymphocytes humains en G0 avec des cellules CHO (Chinese Hamster Ovary) en mitose à l'aide d'un mitogène associée au marquage des télomères et des centromères pour le dénombrement des aberrations chromosomiques sans passer par la phase de culture cellulaire.

Cette technique est très difficile à introduire en routine clinique et ne permet pas la quantification des télomères des lymphocytes humains car l'échantillon contient également les chromosomes des CHO avec une taille des télomères très différente de celle des lymphocytes humains.

Par ailleurs, cet article n'enseigne pas ni ne suggère la quantification globale de la taille des télomères par l'observation de l'intensité de fluorescence totale des noyaux interphasiques.

Zafiroponlous et al. (Int. J. Mod. Phys. Conf. Ser. 2016.44) décrivent également l'utilisation de la technique de « premature chromosome condensation (PCC)» associée au marquage des télomères et des centromères pour le dénombrement des aberrations chromosomiques dans le cadre de dosimétrie biologique de triage après une irradiation accidentelle et le besoin d'avoir la dose absorbée le plus rapidement possible.

Ce document ne décrit pas ni ne suggère la quantification globale des télomères par l'observation de l'intensité de fluorescence totale des noyaux interphasiques.

WO 97/014026 décrit la technique Q-FISH qui consiste à quantifier l'intensité de la fluorescence des télomères dans des cellules en métaphase après le marquage des télomères et centromères par des sondes de type PNA.

CN 108 004 299 décrit une méthode de Q-FISH qui utilise l'ADN génomique au lieu de cellules fixées par méthode cytogénétique (voir paragraphe [0001], [0012], [0013] de ce document). Ce document décrit aussi une sonde PNA spécifique aux télomères et ayant une séquence particulière. Ce document ne décrit pas une sonde pour centromère.

Concernant la détection des aberrations chromosomiques, le marquage homogène des chromosomes demeure la technique conventionnelle la plus utilisée en cytogénétique clinique (classification des chromosomes) comme en toxicologie génétique ou en dosimétrie biologique. Mais cette technique consomme beaucoup de temps et nécessite du personnel hautement qualifié. En effet, cette technique se base sur un prélèvement du sang d'un tube héparine-lithium d'au moins 5 ml. Cet acte nécessite le passage du patient à la salle de prélèvement ou dans un laboratoire d'analyse médicale.

Les approches cytogénétiques, combinées avec l'hybridation in situ par des sondes ADN dirigées contre un chromosome entier ou un gène spécifique, demeurent les approches les plus sensibles et les plus fiables dans le diagnostic des patients mais aussi dans les études des effets clastogènes des produits chimiques. Cependant, cette technique est coûteuse à cause du prix des sondes ADN et le temps d'hybridation.

Par ailleurs, les approches génomiques (Transcriptome, CGH-array, SNP, NGS) ont été proposées comme une alternative dans la détection des altérations du génome. Mais, ces techniques comme les autres approches génomiques, perdent leur sensibilité quand les altérations du génome touchent des séquences répétitives (les séquences télomériques et centromériques).

Par conséquent, il y a toujours une nécessité de développer un procédé à haut débit pour détecter des aberrations du génome touchant à la fois les séquences uniques mais aussi les séquences répétitives en utilisant une quantité très réduite de cellules.

La présente invention a pour objet de proposer un procédé à haut débit permettant de détecter à la fois les aberrations chromosomiques et les aberrations de télomères utilisant un échantillon biologique de 150µl à 200µl, ledit procédé comprenant :
- la préparation d'une lame cytogénétique à partir dudit échantillon dans une microplaque, ladite culture des cellules étant réalisée dans un puits de microplaque, dont le rapport entre la quantité du milieu de culture et la surface du puits est de 1 ml/cm² à 1,5ml/cm², l'indice mitotique dans ladite lame cytogénétique étant 3 fois plus élevé en moyenne que celui de la procédure conventionnelle consistant à cultiver les cellules dans des flasques avec 10 à 20 ml de milieu,
- le marquage simultané des télomères et des centromères par des sondes d'acides nucléiques peptidiques avec un temps d'hybridation de 30 minutes à 1,5 heures.
- la quantification par flux d'images de l'intensité de fluorescence de télomères sur des noyaux interphasiques en utilisant un objectif de grossissement 10x pour une quantification globale des télomères,
- éventuellement, la quantification des micronoyaux et des ponts anaphasiques en utilisant un objectif de grossissement 10x voir 40x, et
- la capture automatique des chromosomes en métaphase pour détecter les aberrations chromosomiques et/ou les aberrations de télomères de chaque chromosome avec un objectif de grossissement 63x.

On entend par « aberration chromosomique », l'anomalie de nombre ou de structure d'un chromosome conduisant à une altération du caryotype. A titre d'exemple d'aberrations chromosomiques de structure, on peut citer le chromosome dicentrique, les anneaux centriques, les chromosomes acentriques, les translocations chromosomiques, l'isochromosome, les insertions et les délétions. A titre d'exemple d'anomalie du nombre de chromosomes, on peut citer les chromosomes surnuméraires.

La méthode de l'invention permet également de détecter les micronoyaux et les ponts anaphasiques, qui font partie des aberrations chromosomiques.

On entend par « micronoyaux » des fragments de chromosomes ou des chromosomes entiers expulsés par le noyau cellulaire au cours de la mitose et formant de petites entités bien individualisées dans le cytoplasme des cellules en interphase.

On entend par « pont anaphasique » de l'ADN sous forme de filaments entre deux cellules filles avec ou sans séquence télomérique et centromérique, un pont anaphasique indique la présence d'un chromosome dicentrique qui est un marqueur important d'instabilité chromosomique ou d'exposition à un agent génotoxique.

On entend par « aberrations de télomères » les aberrations touchant la région contenant la séquence répétitive des télomères qui se situe dans les régions terminales de chromosomes. Comme exemple d'aberrations de télomères, on peut citer la perte d'un télomère, la perte de deux télomères du même bras (délétion) et la formation de dédoublements des télomères.

On entend par « indice mitotique » le taux des cellules en mitose, dans un échantillon cellulaire donné, par rapport au nombre total de cellules.

L'indice mitotique reflète la vitesse de division cellulaire.

On entend par « indice mitotique dans ladite lame cytogénétique étant 3 fois plus élevé en moyenne que celui de la procédure conventionnelle » l'indice mitotique mesuré dans une lame cytogénétique de la présente invention préparée selon la méthode décrite ci-après étant 3 fois plus élevé en moyenne que celui obtenu par une méthode conventionnelle dans un même échantillon provenant d'un même sujet et mise en œuvre par un même praticien avec les mêmes équipements.

On entend par « quantification globale des télomères » la quantification à partir d'images de l'intensité de fluorescence de télomères sur des noyaux interphasiques obtenus avec un objectif de grossissement 10x, pour obtenir les données consistant en :
- la taille moyenne des télomères et la taille médiane des télomères,
- la fréquence des cellules avec des télomères courts,
- les distributions statistiques afin de déterminer les hétérogénéités intercellulaires et interindividuelles,
- l'âge biologique de l'échantillon calculé à partir d'une courbe étalon de donneurs sains.

Les intensités brutes de fluorescence de télomères sur des noyaux interphasiques sont normalisées par rapport à celles obtenues sur des lames témoins et converties en kilobase en utilisant une courbe étalon.

La quantification globale des télomères est effectuée par un logiciel spécifique pour obtenir les susdites données (TeloScore).

On entend par « capture automatique des chromosomes en métaphase » la quantification automatique à partir d'une image de l'intensité de fluorescences des télomères et des centromères des chromosomes en métaphase pour obtenir les données consistant en :
- l'indentification de chaque chromosome à partir de sa taille, ladite taille étant définie par la distance entre les télomères du bras court (p) et ceux du bras long (q) du chromosome ainsi que par le rapport entre la taille de la partie p et la partie q du chromosome (index centromérique),
- la quantification du nombre des centromères, afin d'analyser uniquement les métaphases complètes,
- la quantification du signal de chaque télomère de chaque chromosome en métaphase, soit 4 signaux par chromosome et 184 signaux attendus par métaphase diploïde).

Cette capture automatique permet :
- la classification des chromosomes
- la détection des pertes et des délétions des télomères de chaque chromosome ;
- la détection des aberrations chromosomiques de structure telles que les chromosomes dicentriques, les anneaux centriques, les anneaux acentriques et les différents types de chromosomes acentriques ;
- Pour la détection des remaniements chromosomiques simples, le marquage en bandes DAPI, proche de celui des bandes GTG, complète l'identification des chromosomes
- Pour la détection des remaniements complexes, un marquage de type M-FISH, effectué sur les mêmes métaphases, permet la réalisation d'un caryotype multicolore qui rend l'analyse plus fiable et plus sensible.

L'analyse automatique des chromosomes en métaphase est effectuée par un logiciel spécifique (ChromoScore).

La présente invention est mise en œuvre grâce à la mise au point des techniques suivantes :
- la préparation d'une lame cytogénétique dans laquelle la proportion de cellules en métaphase est supérieure à celle d'une lame cytogénétique conventionnelle;
- la mise au point d'une méthode de marquage des télomères et des centromères qui réduit considérablement le temps de marquage par rapport à une technique conventionnelle nécessitant 4 à 6 heures de travail,
- l'utilisation d'un objectif microscopique de grossissement 10x qui permet de quantifier globalement les télomères dans 10 000 cellules interphasiques en moins de 2 minutes,
- la capture automatique des métaphases permettant de réaliser des marquages successifs sur les mêmes métaphases mais aussi une automatisation dans le dénombrement des aberrations télomériques ou chromosomiques.
- la détection automatique des micronoyaux et des ponts anaphasiques sur des cellules mononuclées sur une même lame cytogénétique.

Le présent procédé présente plusieurs avantages techniques par rapport aux méthodes existantes.

Premièrement, le procédé de l'invention est mis en œuvre avec une quantité très réduite de cellules. Par exemple, il suffit d'utiliser seulement 150µl à 200µl du sang total, autrement dit une quantité du sang qui peut être obtenue en piquant l'index en utilisant le même principe que la mesure de la glycémie. Cette réduction du volume de l'échantillon facilite son prélèvement, économise considérablement le temps d'analyse et les réactifs consommés.

Deuxièmement, contre toute attente, il est observé pour la première fois par l'Inventeur que le rapport surface/volume d'un récipient de culture peut influencer l'indice mitotique et que le rapport surface/volume d'un puits de microplaque est particulièrement favorable pour augmenter cet indice et avoir des cultures très riches et faciles à analyser. L'utilisation d'une microplaque pour la culture de l'échantillon permet non seulement de réduire la quantité d'échantillon nécessaire et le temps de culture, mais permet également de préparer une lame cytogénétique avec un indice mitotique supérieur à celle d'une lame cytogénétique conventionnelle, ce qui permet, d'une part, une étude cytogénétique plus fiable et d'autre part, de réaliser un marquage simultané des télomères et des centromères sur des cellules en interphase et des cellules en métaphase.

Cette approche permet de donner rapidement à la fois des informations relatives à la quantification globale des télomères sur noyaux interphasiques mais aussi des informations sur chaque chromosome afin de détecter des aberrations chromosomiques et les aberrations de télomères en utilisant les métaphases. Le procédé de la présente invention permet, d'une part, d'établir une relation entre la taille des télomères et la fréquence des aberrations chromosomiques sur une même lame, mais également de réaliser aussi le classement des chromosomes (caryotype) à partir des chromosomes en métaphase et de chercher une éventuelle mutation génétique. Elle permet aussi de détecter les ponts anaphasiques indiquant la présence d'un chromosome dicentrique qui est un marqueur important d'instabilité chromosomique ou d'exposition à un agent génotoxique.

### Préparation d'une lame cytogénétique

On entend par « lame cytogénétique » une lame transparente, telle qu'une lame de verre, apte à être observée par microscopie, sur laquelle sont étalées les cellules à analyser et fixés en métaphase et en interphase. L'indice mitotique, qui nous permet d'avoir une idée sur la richesse de la lame en métaphase, a été calculé par la présente invention et par la méthode de cytogénétique conventionnelle sur les mêmes échantillons. Le rapport de l'indice mitotique est compris entre 1,5 et 4 entre une lame cytogénétique préparée par la présente invention et celle préparée par la technique de cytogénétique conventionnelle.

Dans un échantillon préparé selon une méthode conventionnelle, une culture en suspension de cellules est effectuée dans un récipient de volume de 10 à 20 ml.

L'augmentation de l'indice mitotique est corrélée avec l'augmentation de la proportion des cellules en métaphase.

Etant donné que les chromosomes en métaphase sont plus condensés et plus visibles, l'augmentation qualitative et quantitative de la proportion des chromosomes en métaphase permet non seulement d'améliorer la détection des aberrations chromosomiques mais aussi d'automatiser le dénombrement des aberrations chromosomiques.

Dans le cadre de la présente invention, on entend par « échantillons biologiques » des préparations de liquides ou tissus biologiques d'un animal contenant des cellules à analyser. De préférence il s'agit d'échantillons sanguins, de liquide amniotique dans le cas de diagnostic prénatal ou de moelle osseuse pour les hémopathies malignes. De préférence, l'animal est un mammifère, et plus favorablement un humain, quel que soit son sexe ou son âge. Il peut s'agir d'un embryon, d'un fœtus, d'un nouveau-né, d'un enfant, d'un adolescent, ou d'un adulte.

Dans un mode de réalisation avantageux, ledit échantillon biologique est un échantillon du sang total, de la moelle, ou un échantillon de cellules tissulaires.

Ledit échantillon du sang total peut être obtenu en utilisant le même principe que l'obtention de quelques gouttes de sang pour la mesure de la glycémie.

Ledit échantillon de cellules tissulaires peut être obtenu à partir d'un fragment de tissu de nature tumorale ou non tumorale qui peut être prélevé d'un patient par biopsie. Ledit échantillon de cellules tissulaires peut être une culture primaire ou une culture secondaire.

Selon le procédé de la présente invention, l'étape de préparation de la lame cytogénétique comprend :
- la culture des cellules dans un puits de microplaque, le rapport entre la quantité du milieu de culture et la surface du puits étant de 1ml/cm² à 1,5ml/cm².

Plus particulièrement, ladite microplaque peut être une microplaque de 24 puits, dont le volume de travail de chaque puits est de 0,5 ml à 1,5 ml.

La lame cytogénétique préparée à partir de cette culture présente un indice mitotique 3 fois plus élevé qu'une lame préparée par une méthode conventionnelle effectuée par un même praticien en utilisant un même échantillon biologique initial et le même équipement expérimental.

La culture de cellules peut être effectuée avec un milieu de culture liquide conventionnel pendant 72h. Ledit milieu de culture peut être le milieu RPMI1640. Après la culture, les cellules sont traitées par un inhibiteur du fuseau mitotique, par exemple la colchicine, pour bloquer le cycle cellulaire des chromosomes en métaphase.

Les cellules subissent ensuite un choc hypotonique qui entraine le gonflement des cellules par différence de pression osmotique.

Les constituants cellulaires sont ensuite fixés grâce à un fixateur, par exemple un mélange acide acétique/méthanol. La proportion volumique entre l'acide acétique et le méthanol dans un tel mélange peut être 1V/3V.

Les susdits traitements de cellules peuvent être effectués directement dans un puits de microplaque.

### Méthode de marquage

Dans un mode de réalisation avantageux du procédé de la présente invention, le marquage simultané des télomères et des centromères par des sondes d'acides nucléiques peptidiques comprend :
- une seule étape de traitement d'une lame cytogénétique pendant 1 à 3 minutes, en particulier 2 minutes, par une solution de formaldéhyde de 3-5%, en particulier une solution à 4%.

Contrairement à une méthode conventionnelle qui nécessite de mettre en œuvre plusieurs fois la fixation par le formaldéhyde, le procédé de la présente invention nécessite une seule étape de traitement par une solution de formaldéhyde.

Ce traitement permet de fixer la structure tridimensionnelle des chromosomes.

Dans un mode de réalisation plus avantageux du procédé de la présente invention, le marquage simultané des télomères et des centromères par des sondes d'acides nucléiques peptidiques comprend en outre, après l'étape de traitement par le formaldéhyde,
- une seule étape de traitement de la lame cytogénétique par de la pepsine pendant 3-5 minutes, en particulier 4 minutes, par immersion d'une lame cytogénétique préalablement traitée par du formaldéhyde dans une solution de pepsine à une concentration de 0,1-0,2ug/ml.

Le traitement par pepsine permet d'hydrolyser les protéines nucléaires et de favoriser l'accès des sondes.

Dans un mode de réalisation plus avantageux du procédé de la présente invention, le marquage simultané des télomères et des centromères comprend, en outre, après l'étape de traitement par la pepsine, les étapes suivantes :
- la déshydratation de la lame cytogénétique successivement pendant 1 minute par une solution aqueuse d'éthanol à 50%, une solution aqueuse d'éthanol à 70% et de l'éthanol pur,
- la dénaturation de l'ADN chromosomique présent sur la susdite lame cytogénétique obtenue à la fin de l'étape précédente et la dénaturation des sondes d'acides nucléiques peptidiques pour télomères et centromères,
- l'hybridation pendant 10-30 minutes, en particulier 20 minutes, à température ambiante, entre l'ADN chromosomique dénaturé et les sondes d'acides nucléiques peptidiques dénaturés obtenus dans l'étape précédente,
- les lavages successifs de ladite lame cytogénétique après hybridation.

Dans un mode de réalisation particulièrement avantageux du procédé de la présente invention, le marquage simultané des télomères et des centromères par des sondes d'acides nucléiques peptidiques comprend ou consiste en les étapes suivantes :
- le traitement d'une lame cytogénétique pendant 1-3 minutes, en particulier 2 minutes, par une solution formaldéhyde de 3-5%, en particulier une solution de formaldéhyde à 4%,
- le traitement de la lame cytogénétique obtenue à la fin de l'étape précédente par de la pepsine pendant 3-5 minutes, en particulier 4 minutes, par immersion de ladite lame cytogénétique dans une solution de pepsine à une concentration de 0,1-0.2ug/ml,

- la déshydratation de la lame cytogénétique obtenue à la fin de l'étape précédente successivement pendant 1 minute par une solution aqueuse d'éthanol à 50%, une solution aqueuse d'éthanol à 70% et de l'éthanol pur,
- la dénaturation de l'ADN chromosomique présent sur la lame cytogénétique obtenue à la fin de l'étape précédente et la dénaturation des sondes d'acides nucléiques peptidiques pour télomères et centromères,
- l'hybridation pendant 10-30 minutes, en particulier 20 minutes, à température ambiante, entre l'ADN chromosomique dénaturé et les sondes d'acides nucléiques peptidiques dénaturés obtenus dans l'étape précédente,
- les lavages successifs de ladite lame cytogénétique obtenue à la fin de l'étape précédente.

Par rapport à la technique conventionnelle qui nécessite une journée de travail, le marquage simultané des télomères et des centromères avec le procédé de la présente invention peut être réalisé en 1h en gardant une intensité du signal comparable avec celle obtenue par la technique conventionnelle.

On entend par « sonde d'acide nucléique peptidique » un oligomère artificiel similaire à une molécule d'ADN ou d'ARN, dont le squelette est composé d'une répétition d'unités N-(2-aminoéthyl)-glycine reliées par une liaison peptidique, les bases purine et pyrimidine étant attachées au squelette par des liaisons méthylcarbonyles. Ladite sonde possède de 5 à 30 bases nucléiques.

Les séquences des sondes d'acides nucléiques peptidiques sont respectivement complémentaires de la séquence répétitive dans la région télomérique ou de la séquence répétitive dans la région centromérique. Par conséquent, les sondes d'acides nucléiques peptidiques s'hybrident avec les télomères ou les centromères sur ces régions.

Les sondes d'acides nucléiques peptidiques sont marquées respectivement par un fluorochrome, tel que les dérivés de cyanine 3, les dérivés de rhodamine ou les dérivés de fluorescéine. La densité de la fluorescence émise par les sondes d'acides nucléiques peptidiques s'hybridant sur les télomères permet par conséquent de quantifier la longueur des télomères.

L'étape de marquage de la présente invention permet de marquer les télomères et les centromères avec un signal très fort en un temps d'hybridation de 30 minutes à 1,5 heures. Le marquage simultané des télomères et des centromères permet de définir rapidement un chromosome par son centromère et le territoire chromosomique par les télomères, ce qui va rendre le dénombrement des aberrations chromosomiques et le classement des chromosomes plus fiables et plus robustes et indépendants de la personne qui analyse.

Dans un mode de réalisation particulier du procédé de la présente invention, une première sonde d'acides nucléiques peptidiques marque les télomères et une 2^{ème} sonde d'acides nucléiques peptidiques marque les centromères, la première sonde et la 2^{ème} sonde émettant respectivement une fluorescence distinctive.

La séquence de la 1^{ère} sonde d'acide nucléique peptidique est complémentaire de la séquence répétitive dans la région télomérique.

La séquence de la 2^{ème} sonde d'acide nucléique peptidique est complémentaire de la séquence répétitive dans la région centromérique.

### Quantification de la taille des télomères par flux d'images

Contrairement à la quantification du signal fluorescent en cytogénétique qui a toujours été réalisée avec l'objectif microscopique de grossissement 63X, le présent procédé utilise un objectif de grossissement 10x pour la quantification globale des télomères par l'observation de l'intensité de fluorescence totale des noyaux interphasiques, ce qui permet de calculer la taille moyenne et la taille médiane des télomères mais aussi l'hétérogénéité dans l'échantillon. L'observation est effectuée avec un objectif de grossissement 10x pour un grand champ d'observation et une profondeur de champ au moins équivalente à l'épaisseur de l'échantillon. Cela permet d'analyser un grand nombre de cellules en gardant la précision et la sensibilité et en évitant les problèmes de saturation dans la capture d'un signal fluorescent. La rapidité du système d'acquisition et d'analyse rend cette mesure particulièrement intéressante. Avec cette technique, 10 000 cellules pourront être analysées en moins de 2 minutes. Elle offre en plus un histogramme de la fréquence de la longueur des télomères et des données sur la morphologie cellulaire (irrégularités, rondeurs, concavité...) en fonction de la taille des télomères ou de la quantité de 4',6-diamidino-2-phénylindole (DAPI) utilisé pour marquer les chromosomes. Des contrôles internes pourront être introduits à chaque coloration. Il s'agit de deux lignées lymphoblastoïdes avec une caractérisation cytogénétique et télomérique bien établie. La taille des télomères de ces deux lignées a été mesurée par plusieurs autres techniques de quantification des télomères (TRF, PCR and Flow FISH). Ces deux contrôles nous permettent de convertir l'intensité de la fluorescence en unité kilobase. A ce contrôle interne s'ajoute un contrôle externe comportant des billes fluorescentes avec une intensité bien précise. Cette lame de calibration a pour objectif de vérifier l'intensité de fluorescence du système de capture, y compris l'intensité de fluorescence de la lampe. Un ajustement de l'intensité de fluorescence sera réalisé avant chaque capture en ajustant plusieurs paramètres tels que l'intensité de la lampe ou le gain de la caméra (TeloScore)

### Détection de micronoyaux et des ponts anaphasiques :

La détection des micronoyaux et des ponts anaphasiques est effectuée automatiquement par microscopie à fluorescence à l'aide d'un logiciel (BridgeScore) sur cellules mononuclées. Pour les micronoyaux, le marquage des télomères et des centromères permet de déterminer la nature de ces micronoyaux. Les micronoyaux détectés avec uniquement les séquences télomériques correspondent à des délétions terminales liées à une exposition à agent clastogène. Les micronoyaux détectés avec des séquences télomériques et centromériques correspondent à l'élimination d'un chromosome entier qui est liée à une exposition à un agent aneugène.

La détection des ponts anaphasiques concerne la présence d'un filament d'ADN reliant deux cellules filles appelé pont anaphasique. La longueur de ces ponts est mesurée ainsi que la présence ou pas des séquences centromériques ou télomériques. La présence des séquences télomériques ou centromériques dans le pont anaphasique montre que le chromosome dicentrique est issu d'une fusion de deux chromosomes avec un dysfonctionnement télomérique. Il est aussi cherché d'une façon automatique le nombre des cellules qui sont collées et qui pourront signaler une configuration spécifique des chromosomes dicentriques (les deux centromères sont très proches). Dans ce cas, la taille du pont anaphasique est très réduite et les deux cellules filles apparaissent collées comme des cellules binucléées.

### Capture automatique des chromosomes en métaphase

La recherche et la capture des métaphases sont effectuées automatiquement par microscopie à fluorescence à l'aide d'un logiciel avec un temps d'intégration de capture bien déterminé.

Les différentes parties des chromosomes sont marquées par différents fluorochromes. Les chromosomes peuvent être marqués par différents fluorochromes qui se lient chacun fortement à certaines bases nucléiques : par exemple, les centromères sont marqués par FITC porté par une sonde d'acide nucléique peptidique ciblant les centromères, les télomères sont marqués par la cyanine 3 porté par une sonde d'acide nucléique peptidique ciblant les télomères et les squelettes du chromosome sont marqués par le DAPI (4',6-diamidino-2-phénylindole).

Ce marquage du chromosome permet de déterminer une codification de chaque chromosome comme de chaque aberration et la mise au point d'un logiciel.

A l'aide de ce logiciel spécifique, le squelette du chromosome, la distance de chaque télomère par rapport à l'extrémité du squelette (distance D), et la distance entre chaque télomère et le centromère (distance d) sont calculés. Les distances d et D sont utilisées pour identifier les paires des chromosomes à partir de leur taille et aussi du rapport D/d.

Les paramètres tels que, le nombre de centromères, le nombre de télomères détectés par chromosome, le nombre de télomères détectés par cellule, la somme de l'intensité de fluorescence, le moyen de l'intensité fluorescence, le médium de l'intensité fluorescence, le ratio p/q des intensités sont calculés.

Ces informations permettent de classer les chromosomes et d'indiquer les aberrations chromosomiques ou les aberrations de télomères.

Le procédé de la présente invention peut aussi être combiné avec les autres techniques cytogénétiques, tels que la technique M-FISH (multiplex fluorescence in situ hybridization) et NGS (Next-Generation Sequencing)-exome qui permet d'étudier les régions codant pour les protéines du génome. A titre d'exemple, l'association du procédé de la présente invention avec la technique de NGS-exome pourra cibler uniquement les régions codantes du génome impliquées dans les remaniements détectés après marquage des télomères et des centromères suivis d'une coloration M-FISH. Cette approche permet de réaliser un NGS ciblé.

Un autre objet de la présente invention concerne des kits de détection.

L'invention propose un kit de détection à haut débit pour la quantification des télomères et la détection des aberrations chromosomiques et/ou des aberrations de télomères, comprenant :
- une solution prête à l'emploi des sondes d'acides nucléiques peptidiques pour des télomères et des centromères,
- des tampons de lavage nécessaires pour l'hybridation,
- des lames étalons pour la quantification des télomères,
- des lames étalons pour contrôler l'intensité de fluorescence du microscope.

Les lames étalons pour la quantification des télomères sont des lames de référence préparées à partir des lignées lymphoblastoïdes dérivées de sujets sains. La taille des télomères a été mesurée par la technique de référence TRF pour avoir la taille en kilobase. Ces lignées ne comportent aucune aberration chromosomique.

Les lames étalons pour contrôler l'intensité de fluorescence du microscope sont des lames avec des billes fluorescentes calibrées avec une intensité bien définie.

La présente invention concerne également une méthode de détection des aberrations chromosomiques, ladite méthode comprenant :
- le marquage des télomères et des centromères des cellules en métaphase par une solution prête à l'emploi des sondes d'acides nucléiques,
- la capture automatique des signaux fluorescents du marquage des télomères et des centromères,
- l'analyse de l'image obtenue dans l'étape précédente pour obtenir l'ensemble des données : le comptage du nombre des centromères dans la métaphase, l'identification de chaque chromosome à partir de sa taille et du rapport entre le bras court (p) et le bras long (q), et la quantification du signal de chaque télomère de chaque chromosome en métaphase, la détection des aberrations chromosomiques de structure telles que les chromosomes dicentriques, les anneaux centriques, les anneaux acentriques et les différents types de chromosomes acentriques,
- éventuellement le marquage en bandes DAPI, proche de celui des bandes GTG, pour compléter l'identification des chromosomes et détecter les translocations simples,
- éventuellement un marquage de type M-FISH effectué sur les mêmes métaphases, pour la réalisation d'un caryotype multicolores pour détecter les remaniements complexes et rend l'analyse plus fiable et plus simple.

Dans un mode de réalisation, les cellules en métaphase sont celles contenues dans une lame cytogénétique préparée selon la méthode de la présente invention décrite ci-dessus.

La présente invention concerne également une méthode de détection des micronoyaux et des ponts anaphasiques, ladite méthode comprenant :
(i) le marquage des télomères et des centromères des cellules en interphase par une solution prête à l'emploi des sondes d'acides nucléiques,
(ii) la capture automatique des signaux fluorescents du marquage des télomères et des centromères,
(iii) l'analyse de l'image obtenue dans l'étape (ii) pour obtenir l'ensemble des données : le nombre total de micronoyaux, le nombre de micronoyaux avec uniquement des séquences télomériques et le nombre des micronoyaux avec des séquences télomériques et centromériques
(iv) l'analyse de l'image obtenue dans l'étape (ii) pour obtenir l'ensemble des données : l'identification des ponts anaphasiques par la détection de la présence d'un filament d'ADN reliant deux cellules filles, la longueur du pont, la détection de la présence ou pas des séquences centromériques ou télomériques dans le pont, le comptage du nombre des cellules collées (pont très réduit).

Les micronoyaux peuvent être identifiés s'il y a la présence seule des séquences télomériques, qui correspond à des délétions terminales liées à une exposition à un agent clastogène, ou la présence simultanée des séquences télomériques et centromériques, qui correspond à l'élimination d'un chromosome entier qui est liée à une exposition à un agent aneugène.

La présence des ponts anaphasiques est corrélée avec la détection d'un filament d'ADN reliant deux cellules filles. La longueur de ces ponts est mesurée ainsi que la présence ou pas des séquences centromériques ou télomériques dans le pont. La présence de ces séquences dans le pont anaphasique montre que le chromosome dicentrique est issu d'une fusion de deux chromosomes avec un dysfonctionnement télomérique. Le nombre des cellules qui sont collées est aussi cherché et peut signaler une configuration spécifique des chromosomes dicentriques (les deux centromères sont très proches).

Cette méthode peut être effectuée automatiquement par microscopie à fluorescence à l'aide d'un logiciel (BridgeScore).

La présente invention est expliquée davantage par les figures et les exemples ci-après.

### Figures

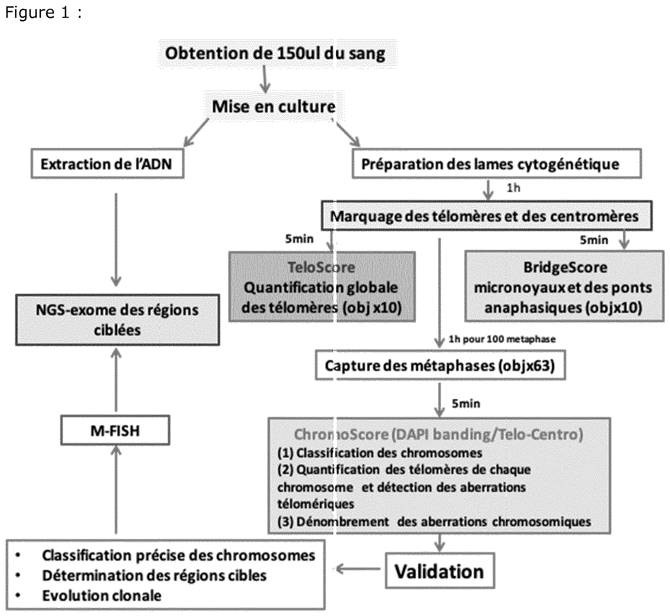

**Figure 1** : cette figure illustre un schéma du procédé à haut débit de quantification des télomères, de détection des micronoyaux et des ponts anaphasiques et de détection des aberrations chromosomiques et télomériques de la présente invention.
**Figure 2** : cette figure montre la comparaison entre la qualité et la quantité des métaphases obtenues par la technique de microplaque et la technique conventionnelle. Images des métaphases issues de la culture conventionnelles (cadre en haut) et images des métaphases issues des cultures en microplaque (cadre en bas). Cette comparaison a été faite dans les mêmes conditions avec le même échantillon cellulaire.
**Figure 3A** **:** cette figure montre la comparaison de l'intensité de fluorescence des billes de 6 µm de calibration mesurée par un objectif microscopique 10x ou un objectif microscopique 63x avec des intensités différentes (100%, 33% et 10%).
**Figure 3B** : cette figure montre la répartition de l'intensité de fluorescence des cellules en quartile mesurée par un objectif microscopique 10x ou un objectif microscopique 63x.
**Figure 4** **:** cette figure montre la comparaison de la quantification des télomères sur des métaphases (Q-FISH) et sur des interphases (présente invention) en utilisant quatre lignées cellulaires.
**Figure 5** : cette figure montre la comparaison entre la quantification des télomères mesurée par le procédé de l'invention et le résultat obtenu par TRF (A) et le résultat obtenu par PCR (B).
**Figure 6** : cette figure montre la corrélation entre la taille moyenne des télomères mesurée par la présente invention et l'âge sur une cohorte de 420 donneurs sains. La taille moyenne est exprimée en kb.
**Figure 7** : reproductibilité du procédé de l'invention avec deux mesures différentes du même échantillon comportant 420 donneurs.
**Figure 8** : cette figure montre la fréquence des micronoyaux chez des donneurs sains et des patients atteints d'un cancer (A) la totalité des micronoyaux. (B) la différence entre les micronoyaux avec seulement des séquences télomériques détectées grâce à la présente invention entre les patients et les donneurs.
**Figure 9** : cette figure montre la détection des ponts anaphasiques dans des cellules en interphase chez des patients atteints d'un cancer. La méthode de l'invention permet de distinguer les ponts anaphasiques assez longs (flèche blanche) avec des séquences télomériques et/ou centromériques correspondant à la présence d'un chromosome dicentrique issue d'une fusion de deux chromosomes ou les ponts anaphasiques très réduits (flèche grise) indiquant la présence d'un chromosome dicentrique avec deux centromères très proches.
**Figure10** : cette figure montre la détection dans un prélèvement prénatal par le procédé de l'invention d'un chromosome marqueur surnuméraire correspondant à un anneau centrique. Cette aberration ne peut pas être détectée par la technique RBG ou la technique M-FISH seule.

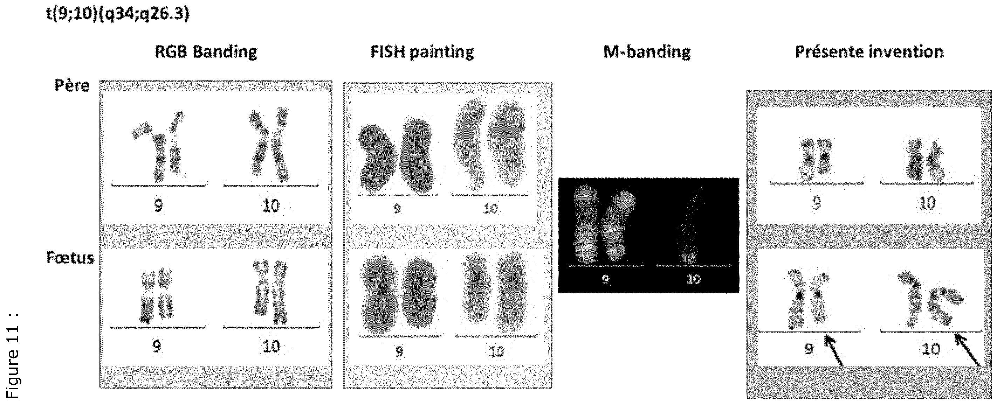

**Figure 11** **:** cette figure montre la détection d'une translocation chromosomique et l'identification de la nature de cette translocation par la technique conventionnelle (figure en haut), par hybridation in situ du chromosome, par M-banding et par le procédé de l'invention (figure en bas) dans le cadre d'un examen prénatal avec décision du conseil génétique sera prise en cas où la translocation n'est pas équilibrée. Comme la translocation touche la partie télomérique du chromosome 10q, elle n'a pas été détectée par la cytogénétique conventionnelle ou moléculaire. La présente invention permet de détecter les séquences télomériques et confirmer la nature de la translocation.
**Figure12****:** cette figure montre la détection des aberrations chromosomiques dans des cellules en métaphase après marquage des télomères et des centromères : les chromosomes dicentriques, les anneaux centriques et les chromosomes acentriques.
**Figure 13** **:** cette figure montre qu'une analyse séquentielle après marquage des télomères et des centromères associés à la technique de M-FISH permet une classification des chromosomes très précise et aussi une détection des aberrations chromosomiques, tels que le chromosome dicentrique avec une configuration très spécifique, une aberration chromosomique difficile à détecter par une technique conventionnelle.

### Exemples

### 1. Matériels et méthodes

### Lignées cellulaires

Les lignées cellulaires testées dans les exemples sont des lignées lymphoblastiques dérivées de donneurs sains, des lignées tumorales du lymphome de Hodgkin, du lymphome de Burkitt et des lymphomes de manteau ont été utilisées.

### Essai Q-FISH

L'essai Q-FISH, qui permet la visualisation des télomères par hybridation en utilisant une sonde de fluorescence sur des métaphases, est mis en œuvre selon la méthode décrite dans [Lansdorp et al. Hum Mol Genet. 1996 May;5(5):685-91.]

### Essai TRF

L'analyse TRF est mise en œuvre selon la technique décrite dans [Kimura et al., Nat Protoc. 2010 Sep;5(9):1596-607]

### Essai qPCR

L'essai qPCR est mis en œuvre selon la méthode décrite dans [OCallaghan & Fenech, Clin Nutr. 2012 Feb;31(1):60-4].

### Marquage des télomères et des centromères

Un protocole de marquage des télomères et des centromères est illustré ci-après.
1. lavage des lames cytogénétiques dans une première solution de tampon phosphate salin (PBS)1 pendant 1 minute à température ambiante,
2. fixation dans une solution de formaldéhyde à 4% pendant 2 minutes à température ambiante,
3. lavage 2 fois dans une solution de tampon phosphate salin (PBS) pendant 1 minute,
4. traitement par une solution de pepsine (0,1ug/ml) à 37°C pendant 4 minutes,
5. lavage 2 fois dans une solution de tampon phosphate salin pendant 1 minute,
6. déshydrations successives pendant 1 minute à 4°C par une solution aqueuse d'éthanol à 50%, une solution aqueuse d'éthanol à 70%, d'éthanol pur
7. séchage des lames,
8. dénaturation des sondes et d'ADN chromosomique à 80°C pendant 3 minutes,
9. hybridation pendant 20 minutes à température ambiante,
10. lavages des lames cytogénétique
11. coloration des chromosomes par le DAPI et application d'un colorant de contraste.

### Analyse des aberrations chromosomiques

L'analyse est effectuée à partir des cellules en métaphase après le marquage des télomères et des centromères et la capture automatique des signaux fluorescents des télomères et des centromères :
- L'identification de chaque chromosome à partir de sa taille définie par la distance entre les télomères des bras courts (p) et des bras longs (q) du chromosome ainsi que par le rapport entre la taille de la partie p et la partie q du chromosome (index centromérique).
- La quantification du nombre des centromères pour analyser uniquement les métaphases complètes.
- La quantification du signal de chaque télomère (4 signaux par chromosome et 184 signaux attendus par métaphase diploïde).
- La détection des pertes et des délétions des télomères de chaque chromosome.
- La détection des aberrations chromosomiques de structure telles que les chromosomes dicentriques, les anneaux centriques, les anneaux acentriques et les différents types de chromosomes acentriques.
- Pour la détection des remaniements chromosomiques simples, le marquage en bandes DAPI, proche de celui des bandes GTG, complète l'identification des chromosomes.
- Pour les remaniements complexes, un marquage de type M-FISH, effectué sur les mêmes métaphases, permet la réalisation d'un caryotype multicolores qui rend l'analyse plus fiable et plus sensible.
- Ce logiciel propose une interface simple permettant une correction manuelle des données et est capable « d'apprentissage » au cours du temps.

### 2. Résultats

### 2.1 Validation technique du procédé de l'invention

### Validation de l'approche culture sur microplaque

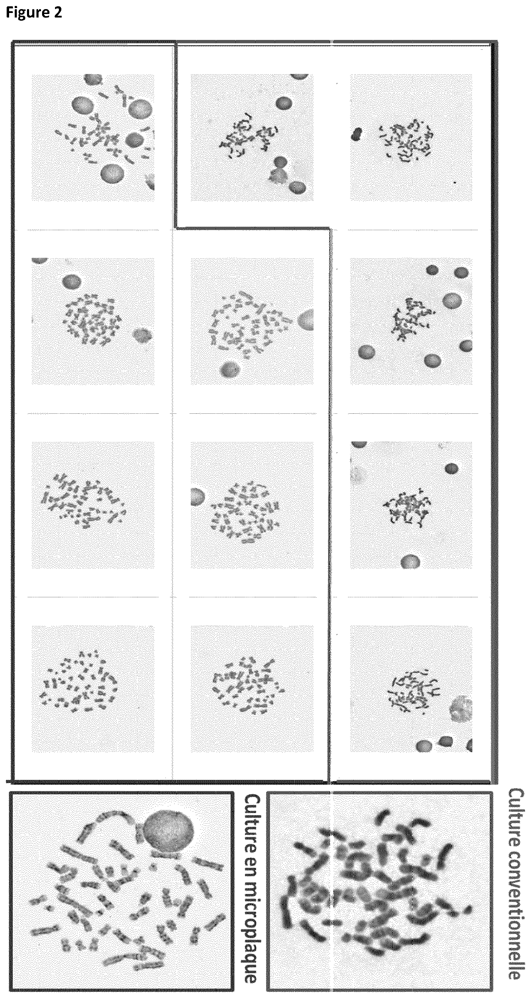

La culture cellulaire en microplaque a été validée par rapport à la technique conventionnelle (culture en flasque de 25cm²) sur une cohorte de 70 patients atteints de pathologies lymphoïdes, 50 donneurs sains et 150 patients atteints d'un syndrome inflammatoire ou prolifératif. Du sang total de 50 patients et de la moelle de 20 patients ont été mis en culture avec les deux approches. Aucun échec de culture n'a été observé avec la technique de microplaque et avec une qualité des métaphases permettant une analyse cytogénétique fiable et sensible. Par contre, à l'aide de la technique conventionnelle, plusieurs échecs de culture ont été observés (5 moelles sur 20 et 7 sang total sur 50 patients cancéreux) avec une qualité des métaphases moins intéressante et qui ne permettra pas une analyse automatique des aberrations chromosomiques (figure 2).

### Capture de noyau interphasique par un objectif de grossissement 10x

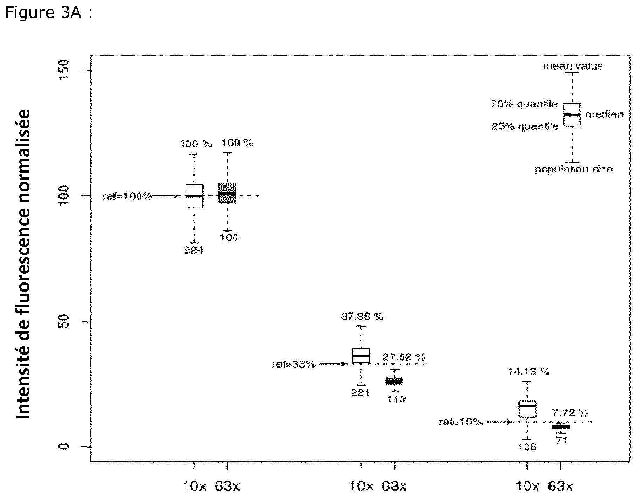

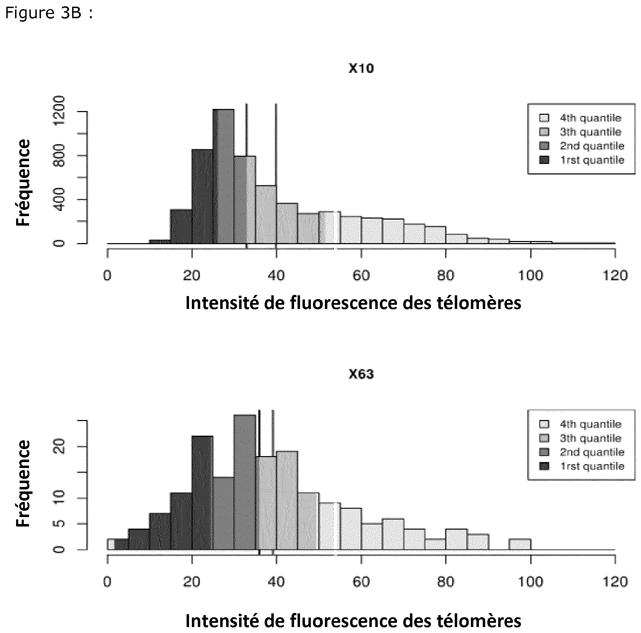

La méthode de capture de la fluorescence par un objectif de grossissement 10x est comparée à une méthode qui utilise un objectif de grossissement 63x. Les intensités de fluorescence émise par les perles calibrées sont respectivement mesurées par un objectif microscopique 10x et un objectif microscopique 63x. Les résultats obtenus sont comparables (figures 3A et 3B). Cependant, pour les faibles intensités fluorescentes, l'utilisation de l'objectif 10x permet de se rapprocher de la valeur exacte de fluorescence des billes.

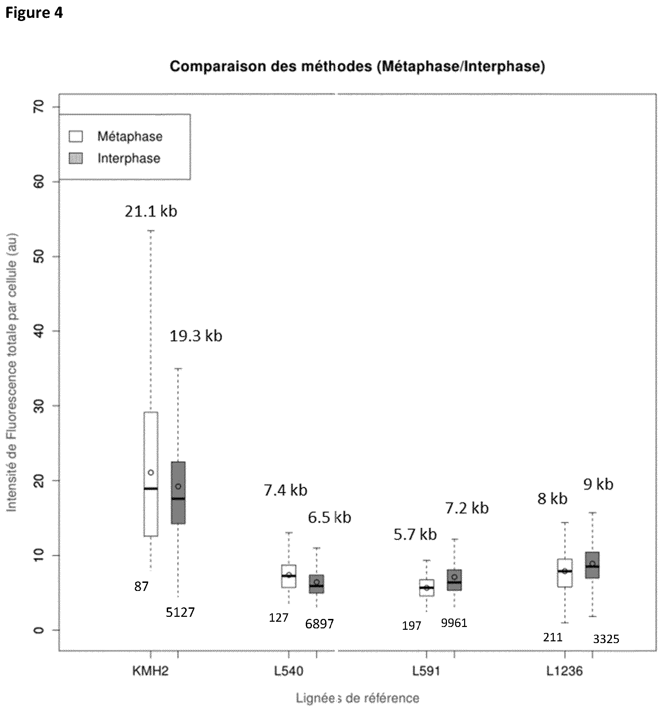

La méthode de capture de noyau interphasique par un objectif de grossissement 10x et la méthode classique (Q-FISH) de capture des métaphases utilisant un objectif de grossissement 63x sont aussi testées sur différentes lignées cellulaires humaines et sur les lymphocytes circulants des patients atteints de cancer. On observe une forte corrélation entre l'intensité de fluorescence obtenue à partir des métaphases par la méthode Q-FISH et l'intensité de fluorescence obtenue à partir de noyaux interphasiques par le procédé de la présente invention (figure 4). Les analyses de la taille des télomères sont réalisées à partir d'un logiciel permettant de donner la taille moyenne, la fréquence des cellules avec des télomères courts et l'hétérogénéité de la taille des télomères.

### Quantification de télomères

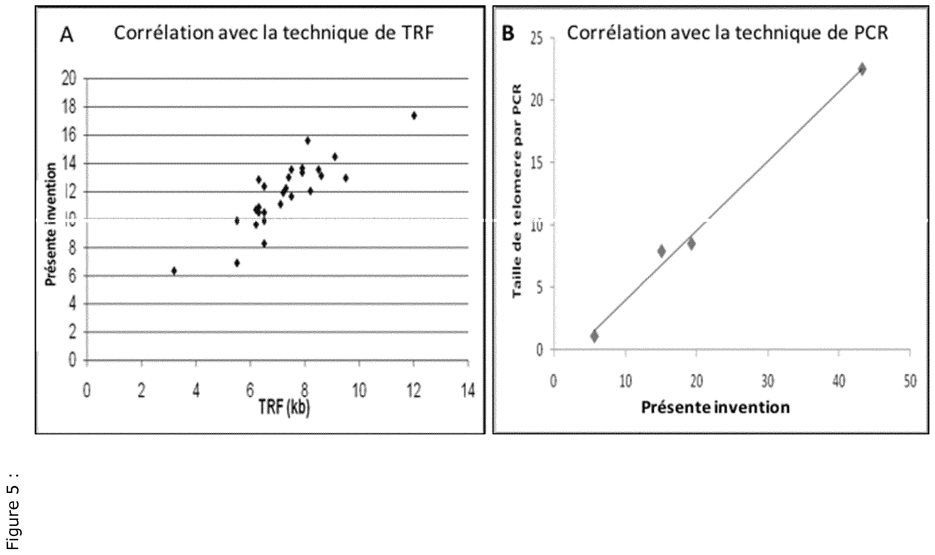

Le résultat de quantification de télomères obtenu par le procédé de la présente invention est aussi comparé avec celui obtenu par la technique TRF (terminal restricted fragment) et celui obtenu par la technique PCR (figure 5). Cette comparaison montre une corrélation significative entre le procédé de la présente invention et les techniques conventionnelles en ce qui concerne la quantification de télomères et valide ainsi cette nouvelle approche par rapport à la technique de référence (TRF) et aussi par rapport à la technique la plus utilisé actuellement (PCR).

### 2.2 Application du procédé de l'invention

### Impact de l'âge sur la taille des télomères

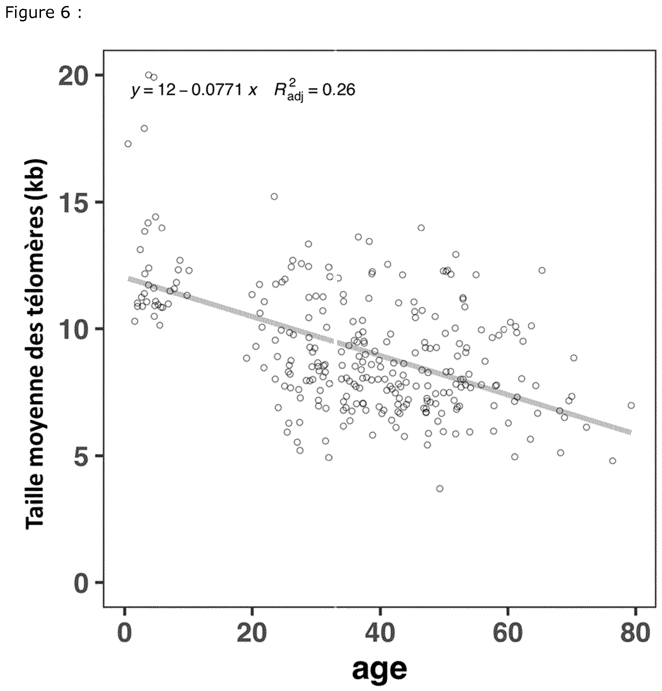

Les tailles des télomères dans les lymphocytes circulants obtenus de 420 donneurs sains âgés de 1 à 80 ans ont été analysées par le procédé de l'invention. Les donneurs sont classés en 5 groupes d'âge. L'intensité de fluorescence totale des télomères est quantifiée. Pour chaque échantillon, plus de 10 000 cellules sont analysées. Le résultat obtenu montre clairement une diminution progressive de la longueur des télomères avec l'augmentation de l'âge avec une perte en moyenne de 76pb chaque année (figure 6). Cette moyenne de régression des télomères en fonction de l'âge est en parfaite corrélation avec la littérature (Vera el al. (2012), Cell Rep 2(4):732-737. ).

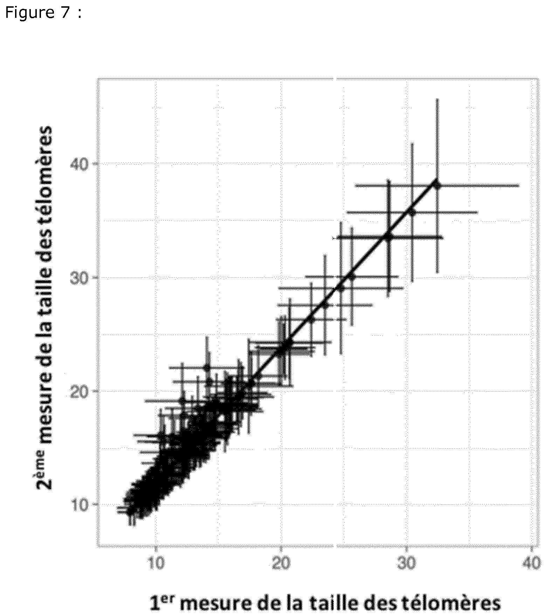

Par ailleurs, le procédé de l'invention donne une bonne reproductibilité avec deux mesures de la taille des télomères de la même population à deux ans d'intervalle (figure 7).

### Fréquence des micronoyaux et des ponts anaphasiques

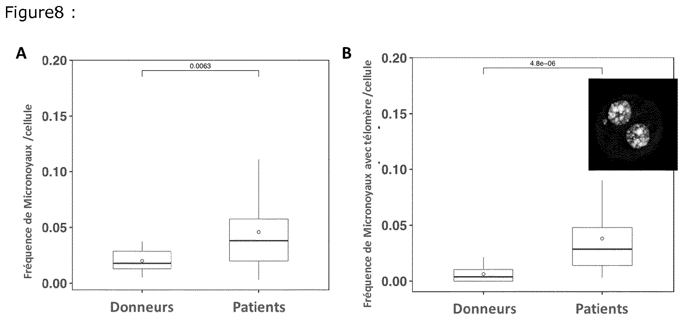

La fréquence des micronoyaux et des ponts anaphasiques a été évaluée dans les lymphocytes circulants des donneurs sains et des patients atteints d'un cancer par le procédé de l'invention. Les résultats obtenus montrent une augmentation significative de la fréquence des micronoyaux essentiellement des micronoyaux avec uniquement des séquences télomériques (Figure 8). La présente invention a permis de détecter la nature de ces micronoyaux qui est liée à un stress génotoxique.

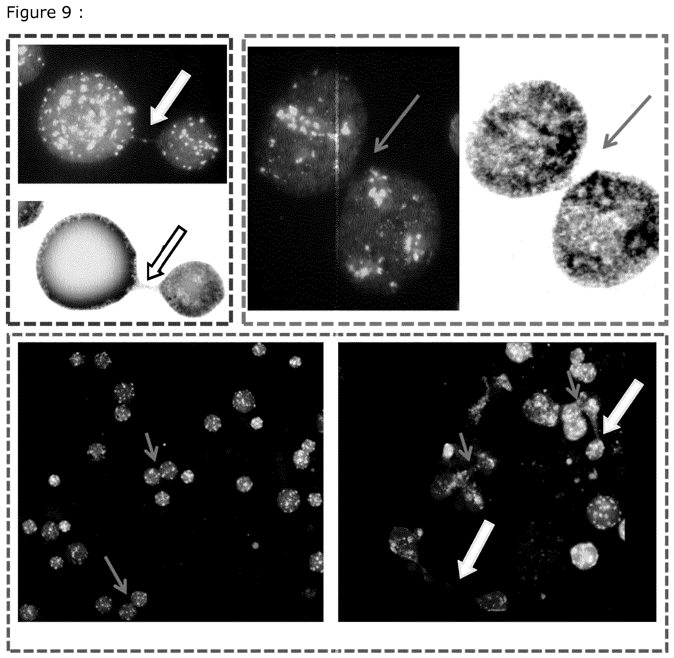

La détection des ponts anaphasiques a été aussi réalisée sur une large cohorte des donneurs sains et des patients. La détection des différentes configurations des ponts anaphasiques a été confrontée aux données obtenues par les aberrations chromosomiques. Le procédé de l'invention permet de détecter les mécanismes impliqués dans la formation des ponts anaphasiques (figure9). La méthode de l'invention permet de détecter les longs ponts anaphasiques, facilement détectable sur lames cytogénétiques et qui sont liés à la formation d'un dicentrique issue d'une fusion de deux chromosomes (dysfonctionnement des télomères). Ce procédé permet aussi de détecter les ponts anaphasiques très réduits qui sont liées à la présence d'une configuration très spécifique des chromosomes dicentriques (les deux centromères très proches). Ces différentes configurations des ponts anaphasiques sont facilement détectables sur les lames cytogénétiques préparées selon la méthode de l'invention décrite ci-dessus permettant une détection très rapide de l'instabilité chromosomique.

### Précision dans la détection des aberrations chromosomiques

### Exemple 1

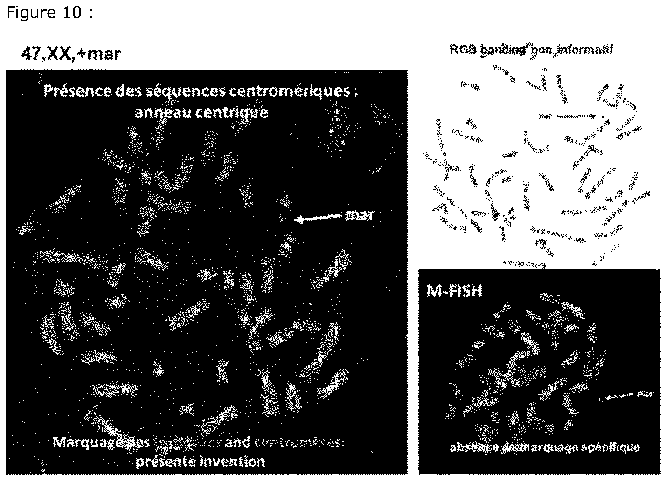

Un chromosome marqueur surnuméraire correspondant à un anneau centrique, essentiellement constitué de séquences centromériques, peut être détecté par le procédé de l'invention. Le phénotype est une infertilité. Cette aberration chromosomique est indétectable par la technique conventionnelle comme la cytogénétique moléculaire ou la technique de M-FISH seule (figure 10). Le marquage des télomères et des centromères a permis de voir des séquences centromériques et de définir la nature de l'aberration chromosomique.

### Exemple 2

En diagnostic prénatal, présence d'une translocation t(9;10)(q34;q26.3) chez le père et le fœtus, la technique conventionnelle (RBG) comme les techniques de cytogénétiques moléculaires (M-Banding et chromosome painting) détectent une translocation non-équilibrée.

Le procédé de l'invention qui conduit au marquage des télomères et des centromères a permis d'observer que le chromosome 10 n'a échangé que la partie télomérique avec le chromosome 9 (figure 11). La présence des séquences télomériques dans le chromosome 9 a permis de montrer qu'il s'agit par conséquent d'une translocation équilibrée. Par rapport aux techniques classiques, le procédé de l'invention permet non seulement de détecter l'aberration chromosomique, mais aussi avec plus de précision la nature de l'aberration.

### Exemple 3

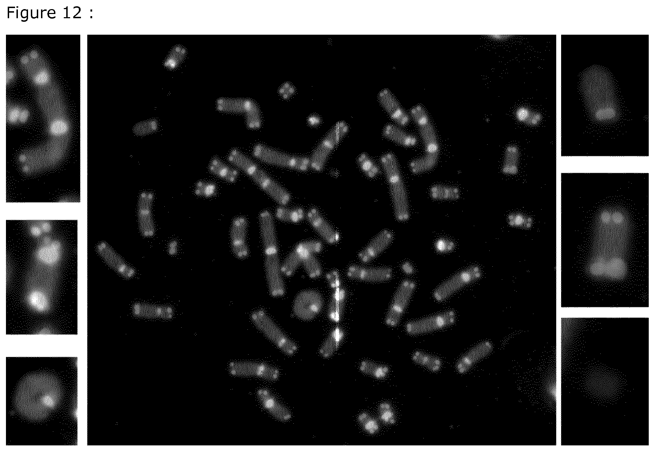

Le procédé de la présente invention permet aussi de détecter toutes les aberrations chromosomiques induites par un agent génotoxique tel que l'irradiation. Le procédé de l'invention permet une évaluation du nombre des centromères, la présence du chromosome dicentrique, des translocations, des différents types des chromosomes acentriques issus d'une délétion terminale, délétion interstitielle ou fusion entre deux délétions terminales. La figure 12 montre que la méthode de l'invention permet de détecter les différentes configurations du chromosome dicentrique, telle que le dicentrique avec les deux centromères linéaires et bien espacés, le dicentrique avec les centromères confondus avec les télomères et le dicentrique avec les deux centromères très proches ou confondus. Cette figure montre que la méthode de l'invention peut aussi faire la distinction entre les anneaux centriques et les anneaux acentriques et faire la caractérisation des chromosomes acentriques, telle que l'acentrique issu d'une délétion terminale, l'acentrique issu d'une délétion interstitielle et l'acentrique issu d'une fusion entre deux acentriques terminaux.

### Exemple 4

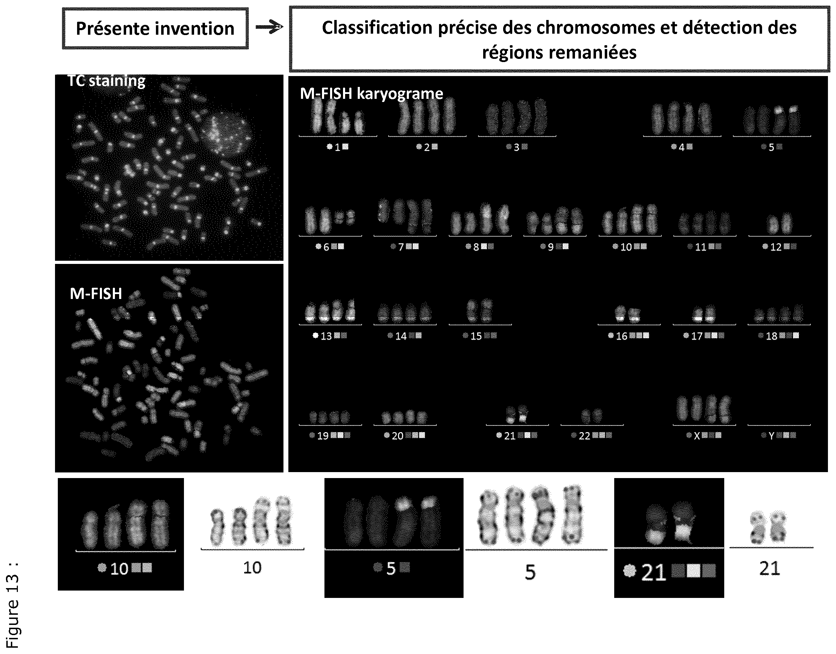

Le procédé de la présente invention permet aussi de détecter une configuration spécifique du chromosome dicentrique, une configuration, qui est très difficilement détectée par les techniques conventionnelles. Le procédé de l'invention permet une classification des chromosomes plus précise et une détection des aberrations chromosomiques plus fiable (figure 13).

### Références :

Benetos A, Toupance S, Gautier S, Labat C, Kimura M, Rossi PM, Settembre N, Hubert J, Frimat L, Bertrand B, Boufi M, Flecher X, Sadoul N, Eschwege P, Kessler M, Tzanetakou IP, Doulamis IP, Konstantopoulos P, Tzani A, Korou M, Gkogkos A, Perreas K, Menenakos E, Samanidis G, Vasiloglou-Gkanis M, Kark JD, Malikov S, Verhulst S, Aviv A. Short Leukocyte Telomere Length Precedes Clinical Expression of Atherosclerosis: The Blood-and-Muscle Model. Circ Res. 2018 Feb 16;122(4):616-623. doi: 10.1161/CIRCRESAHA.117.311751. Epub 2017 Dec 14.
Staerk L, Wang B, Lunetta KL, Helm RH, Ko D, Sherer JA, Ellinor PT, Lubitz SA, McManus DD, Vasan RS, Benjamin EJ, Trinquart L. Association Between Leukocyte Telomere Length and the Risk of Incident Atrial Fibrillation: The Framingham Heart Study. J Am Heart Assoc. 2017 Nov 14;6(11).
Allshire RC(1), Dempster M, Hastie ND Human telomeres contain at least three types of G-rich repeat distributed non -randomly. Nucleic Acids Res. 1989 Jun 26;17(12):4611-27.
Canela A, Vera E, Klatt P, Blasco MA. High-throughput telomere length quantification by FISH and its application to human population studies. Proc Natl Acad Sci U S A. 2007 Mar 27;104(13):5300-5. Epub 2007 Mar 16.
Harley CB(1), Futcher AB, Greider CW. Telomeres shorten during ageing of human fibroblasts. Nature. 1990 May 31;345(6274):458-60.
Kimura M, Stone RC, Hunt SC, Skurnick J, Lu X, Cao X, Harley CB, Aviv A. Measurement of telomere length by the Southern blot analysis of terminal restriction fragment lengths. Nat Protoc. 2010 Sep;5(9):1596-607.
Cawthon RM. Telomere measurement by quantitative PCR. Nucleic Acids Res. 2002 May 15;30(10):e47.
Cawthon RM. Telomere length measurement by a novel monochrome multiplex quantitative PCR method. Nucleic Acids Res. 2009 Feb;37(3):e21.
O'Callaghan NJ(1), Toden S, Bird AR, Topping DL, Fenech M, Conlon MA. Colonocyte telomere shortening is greater with dietary red meat than white meat and is attenuated by resistant starch. Clin Nutr. 2012 Feb;31(1):60-4.
Vera E, Bernardes de Jesus B, Foronda M et al. The rate of increase of short telomeres predicts longevity in mammals. Cell Rep 2012; 2(4):732-737.

## Revendications

1. Procédé à haut débit pour détecter des aberrations chromosomiques et/ou des aberrations de télomères, comprenant :
- la préparation d'une lame cytogénétique à partir d'un échantillon biologique de 150µl à 200µl, cultivé dans une microplaque, ladite culture des cellules étant réalisée dans un puits de microplaque, dont le rapport entre la quantité du milieu de culture et la surface du puits est de 1ml/cm² à 1,5ml/cm², et l'obtention dans ladite lame cytogénétique d'un indice mitotique entre 1,5 et 4 fois plus élevé que l'indice mitotique dans une lame cytogénétique préparée après étalement du même échantillon biologique cultivé dans un récipient de 10 à 20 ml,
- le marquage simultané des télomères et des centromères par des sondes d'acides nucléiques peptidiques avec un temps d'hybridation de 30 minutes à 1,5 heures,
- la quantification par flux d'images de l'intensité de fluorescence de télomères sur des noyaux interphasiques en utilisant un objectif de grossissement 10x pour une quantification globale des télomères, et
- la capture automatique des chromosomes en métaphase pour détecter les aberrations chromosomiques et/ou les aberrations de télomères de chaque chromosome.

2. Procédé selon la revendication 1, **caractérisé en ce que** lesdites aberrations chromosomiques sont choisies parmi : les micronoyaux, les ponts anaphasiques, les chromosomes dicentriques, les anneaux centriques, les chromosomes acentriques, les translocations chromosomiques, l'isochromosome, les insertions et les délétions de chromosomes, et **en ce que** lesdites aberrations de télomères sont choisies parmi : la perte d'un télomère, la perte de deux télomères du même bras et la formation de dédoublement d'un télomère.

3. Procédé selon la revendication 1 ou 2, ledit procédé comprenant en outre :
- la quantification des micronoyaux et des ponts anaphasiques en utilisant un objectif de grossissement 10x, ou un grossissement 40x.

4. Procédé selon l'une quelconque des revendications 1 à 3, **caractérisé en ce que** ledit échantillon biologique est un échantillon de sang total, de la moelle, ou un échantillon de cellules tissulaires.

5. Procédé selon l'une quelconque des revendications 1 à 4, dans lequel le marquage simultané des télomères et des centromères comprend :
- une seule étape de traitement d'une lame cytogénétique pendant 1 à 3 minutes, en particulier 2 minutes par une solution de formaldéhyde de 3-5%, en particulier une solution à 4% de formaldéhyde.

6. Procédé selon la revendication 5, dans lequel le marquage simultané des télomères et des centromères comprend en outre, après le traitement par le formaldéhyde,
- une seule étape de traitement de la lame cytogénétique par de la pepsine pendant 3-5 minutes, en particulier 4 minutes, par immersion d'une lame cytogénétique préalablement traitée par du formaldéhyde dans une solution de pepsine à une concentration de 0,1-0,2ug/ml.

7. Procédé selon la revendication 6, dans lequel le marquage simultané des télomères et des centromères comprend, en outre, après l'étape de traitement par la pepsine, les étapes suivantes :
- la déshydratation de la lame cytogénétique successivement pendant 1 minute par une solution aqueuse d'éthanol à 50%, une solution aqueuse d'éthanol à 70% et de l'éthanol pur,
- la dénaturation de l'ADN chromosomique présent sur la susdite lame cytogénétique obtenue à la fin de l'étape précédente et la dénaturation des sondes d'acides nucléiques peptidiques pour télomères et centromères,
- l'hybridation pendant 10-30 minutes, en particulier 20 minutes, à température ambiante, entre l'ADN chromosomique dénaturé et les sondes d'acides nucléiques peptidiques dénaturés obtenus dans l'étape précédente,
- les lavages successifs de ladite lame cytogénétique après hybridation.

8. Procédé selon l'une quelconque des revendications précédentes, **caractérisé en ce qu'**une première sonde d'acides nucléiques peptidiques marque les télomères et une 2^{ème} sonde d'acides nucléiques peptidiques marque simultanément les centromères, la première sonde et la 2^{ème} sonde émettant respectivement une fluorescence distinctive.

9. Procédé selon l'une quelconque des revendications précédentes pour détecter les aberrations chromosomiques, ledit procédé comprenant :
- le marquage des télomères et des centromères des cellules en métaphase par une solution prête à l'emploi des sondes d'acides nucléiques,
- la capture automatique des signaux fluorescents du marquage des télomères et des centromères,
- l'analyse de l'image obtenue dans l'étape précédente pour obtenir l'ensemble des données : le comptage du nombre des centromères dans la métaphase, l'identification de chaque chromosome à partir de sa taille et du rapport entre le bras court (p) et le bras long (q), la quantification du signal de chaque télomère de chaque chromosome en métaphase, pour la détection des aberrations chromosomiques de structure telles que les chromosomes dicentriques, les anneaux centriques, les anneaux acentriques et les différents types de chromosomes acentriques,
- éventuellement le marquage en bandes DAPI, pour compléter l'identification des chromosomes et détecter les translocations simples,
- éventuellement le marquage de type M-FISH effectué sur les mêmes métaphases, pour la réalisation d'un caryotype multicolore pour détecter les remaniements complexes.

10. Procédé selon l'une quelconque des revendications précédentes pour détecter les micronoyaux et les ponts anaphasiques, ledit procédé comprenant :
(i) le marquage des télomères et des centromères des cellules en interphase par une solution prête à l'emploi des sondes d'acides nucléiques,
(ii)la capture automatique des signaux fluorescents du marquage des télomères et des centromères,
(iii) l'analyse de l'image obtenue dans l'étape (ii) pour obtenir l'ensemble des données : le nombre total de micronoyaux, le nombre de micronoyaux avec uniquement des séquences télomériques et le nombre des micronoyaux avec des séquences télomériques et centromériques,
(iv) l'analyse de l'image obtenue dans l'étape (ii) pour obtenir l'ensemble des données : l'identification des ponts anaphasiques par la détection de la présence d'un filament d'ADN reliant deux cellules filles, la longueur du pont, la détection de la présence ou pas des séquences centromériques ou télomériques dans le pont, le comptage du nombre des cellules collées.

11. Utilisation d'un kit de détection à haut débit pour la quantification des télomères et la détection des aberrations chromosomiques et/ou des aberrations de télomères selon l'une des revendications 1 à 10, ledit kit comprenant :
- une solution prête à l'emploi des sondes d'acides nucléiques peptidiques pour des télomères et des centromères,
- des tampons de lavage nécessaires pour l'hybridation,
- des lames étalons pour la quantification des télomères,
- des lames étalons pour contrôler l'intensité de fluorescence du microscope.

## Patentansprüche

1. Hochdurchsatzverfahren zum Nachweis von Chromosomenaberrationen und/oder Telomeraberrationen, umfassend:
- die Herstellung eines cytogenetischen Objektträgers aus einer biologischen Probe von 150 µl bis 200 µl, die in einer Mikrotiterplatte kultiviert wurde, wobei die Kultur der Zellen in einem Well einer Mikrotiterplatte realisiert wurde, wobei das Verhältnis der Menge des Kulturmediums zur Oberfläche des Well 1 ml/cm² bis 1,5 ml/cm² beträgt und die Gewinnung eines Mitoseindex in dem cytogenetischen Objektträger vom 1,5- bis vierfachen des Mitoseindex in einem cytogenetischen Objektträger, der nach Ausbreitung derselben biologischen Probe, die in einem Gefäß von 10 bis 20 ml kultiviert wurde, präpariert wurde,
- die gleichzeitige Markierung der Telomere und der Centromere durch peptidische Nucleinsäuresonden mit einer Hybridisierungszeit von 30 Minuten bis 1,5 Stunden,
- die Bildflussquantifizierung der Intensität der Telomerfluoreszenz an Interphasekernen unter Verwendung eines Objektivs mit 10-facher Vergrößerung für eine globale Quantifizierung der Telomere und
- die automatische Erfassung von Chromosomen in der Metaphase zum Nachweis von Chromosomenaberrationen und/oder Telomeraberrationen bei jedem Chromosom.

2. Verfahren gemäß Anspruch 1, **dadurch gekennzeichnet, dass** die Chromosomenaberrationen ausgewählt sind aus: Mikrokernen, anaphasischen Brücken, dizentrischen Chromosomen, zentrischen Ringen, azentrischen Chromosomen, Chromosomentranslokationen, dem Isochromosom, Chromosomeninsertionen und -deletionen, und dadurch, dass die Telomeraberrationen ausgewählt sind aus: dem Verlust eines Telomers, dem Verlust von zwei Telomeren desselben Arms und der Bildung einer Spaltung eines Telomers.

3. Verfahren gemäß Anspruch 1 oder 2, wobei das Verfahren weiterhin umfasst:
- die Quantifizierung der Mikrokerne und der anaphasischen Brücken unter Verwendung eines Objektivs mit 10-facher Vergrößerung oder mit 40-facher Vergrößerung.

4. Verfahren gemäß einem der Ansprüche 1 bis 3, **dadurch gekennzeichnet, dass** die biologische Probe eine Vollblutprobe, Knochenmarkprobe oder eine Probe von Gewebezellen ist.

5. Verfahren gemäß einem der Ansprüche 1 bis 4, wobei die gleichzeitige Markierung der Telomere und der Centromere Folgendes umfasst:
- einen einzelnen Schritt der Behandlung eines cytogenetischen Objektträgers während 1 bis 3 Minuten, insbesondere 2 Minuten, mit einer Lösung mit 3-5% Formaldehyd, insbesondere einer Lösung mit 4% Formaldehyd.

6. Verfahren gemäß Anspruch 5, wobei die gleichzeitige Markierung der Telomere und der Centromere nach der Behandlung mit Formaldehyd weiterhin Folgendes umfasst:
- einen einzelnen Schritt der Behandlung des cytogenetischen Objektträgers mit Pepsin während 3-5 Minuten, insbesondere 4 Minuten, durch Eintauchen eines cytogenetischen Objektträgers, der zuvor mit Formaldehyd behandelt worden war, in einer Pepsinlösung mit einer Konzentration von 0,1-0,2 µg/ml.

7. Verfahren gemäß Anspruch 6, wobei die gleichzeitige Markierung der Telomere und der Centromere nach dem Schritt der Behandlung mit Pepsin weiterhin die folgenden Schritte umfasst:
- die sukzessive Entwässerung des cytogenetischen Objektträgers während 1 Minute durch eine 50%-ige wässrige Ethanollösung, eine 70%-ige wässrige Ethanollösung und reines Ethanol,
- die Denaturierung der chromosomalen DNA, die auf dem cytogenetischen Objektträger vorhanden ist, der am Ende des vorigen Schritts erhalten wurde, und die Denaturierung der peptidischen Nucleinsäuresonden für Telomere und Centromere,
- die Hybridisierung während 10-30 Minuten, insbesondere 20 Minuten, bei Raumtemperatur zwischen der denaturierten chromosomalen DNA und den denaturierten peptidischen Nucleinsäuresonden, die im vorigen Schritt erhalten wurden,
- die sukzessive Waschung des cytogenetischen Objektträgers nach der Hybridisierung.

8. Verfahren gemäß einem der vorstehenden Ansprüche, **dadurch gekennzeichnet, dass** eine erste peptidische Nucleinsäuresonde die Telomere markiert und eine zweite peptidische Nucleinsäuresonde gleichzeitig die Centromere markiert, wobei die erste Sonde und die zweite Sonde jeweils eine unterschiedliche Fluoreszenz zeigen.

9. Verfahren gemäß einem der vorstehenden Ansprüche zum Nachweisen von Chromosomenaberrationen, wobei das Verfahren umfasst:
- die Markierung der Telomere und der Centromere der in Metaphase befindlichen Zellen durch eine gebrauchsfertige Lösung der Nucleinsäuresonden,
- die automatische Erfassung der Fluoreszenzsignale der Markierung der Telomere und der Centromere,
- die Analyse des im vorigen Schritt erhaltenen Bilds zur Gewinnung der folgenden Datenmenge: die Anzahl der Centromere in der Metaphase, die Identifizierung jedes Chromosoms anhand seiner Größe und anhand des Verhältnisses zwischen dem kurzen Arm (p) und dem langen Arm (q), die Quantifizierung des Signals von jedem Telomer jedes in Metaphase befindlichen Chromosoms, zum Nachweis von strukturellen Chromosomenaberrationen wie dizentrischen Chromosomen, zentrischen Ringen, azentrischen Ringen und den verschiedenen Typen von azentrischen Chromosomen,
- gegebenenfalls die Markierung in DAPI-Streifen zum Abschließen der Identifizierung der Chromosomen und zum Nachweis der einfachen Translokationen,
- gegebenenfalls eine Markierung der Art M-FISH, die an denselben Metaphasen durchgeführt wird, zur Realisierung eines mehrfarbigen Karyotyps, um die komplexen Umlagerungen nachzuweisen.

10. Verfahren gemäß einem der vorstehenden Ansprüche zum Nachweisen von Mikrokernen und von anaphasischen Brücken, wobei das Verfahren umfasst:
(i) die Markierung der Telomere und der Centromere der in Interphase befindlichen Zellen durch eine gebrauchsfertige Lösung der Nucleinsäuresonden,
(ii) die automatische Erfassung der Fluoreszenzsignale der Markierung der Telomere und der Centromere,
(iii) die Analyse des in Schritt (ii) erhaltenen Bilds zur Gewinnung der folgenden Datenmenge: die Gesamtzahl der Mikrokerne, die Anzahl der Mikrokerne mit ausschließlich Telomersequenzen und die Anzahl der Mikrokerne mit Telomersequenzen und Centromersequenzen,
(iv) die Analyse des in Schritt (ii) erhaltenen Bilds zur Gewinnung der folgenden Datenmenge: die Identifizierung der anaphasischen Brücken durch den Nachweis der Anwesenheit eines DNA-Strangs, der zwei Tochterzellen miteinander verbindet, die Länge der Brücke, der Nachweis der An- oder Abwesenheit von Centromer- oder Telomersequenzen in der Brücke, die Anzahl der verklebten Zellen.

11. Verwendung eines Kits zum Hochdurchsatznachweis zur Quantifizierung der Telomere und zum Nachweis der Chromosomenaberrationen und/oder Telomeraberrationen gemäß einem der Ansprüche 1 bis 10, wobei der Kit umfasst:
- eine gebrauchsfertige Lösung von peptidischen Nucleinsäuresonden für Telomere und Centromere,
- Waschpuffer, die für die Hybridisierung notwendig sind,
- Standardobjektträger zur Quantifizierung der Telomere,
- Standardobjektträger zur Steuerung der Intensität der Fluoreszenz des Mikroskops.

## Claims

1. A high throughput method for detecting chromosomal aberrations and/or telomere aberrations, comprising:
- preparing a cytogenetic slide from a biological sample of 150µL to 200µL, grown in a microplate, the said growing of cells being carried out in a microplate well, wherein the ratio of the amount of the culture medium to the well's surface area is 1mL/cm² to 1.5mL/cm², the mitotic index in said cytogenetic slide being between 1.5 and 4 times higher than the conventional procedure of culturing cells in flasks with 10 to 20 mL of medium,
- simultaneously labelling the telomeres and centromeres with peptide nucleic acid probes with a hybridisation time from 30 minutes to 1.5 hours,
- flow image quantifying the fluorescence intensity of telomeres on interphase nuclei using a 10x magnification objective for overall telomere quantification, and
- automatically capturing the metaphase chromosomes to detect chromosomal aberrations and/or telomere aberrations in each chromosome.

2. The method according to claim 1, wherein said chromosomal aberrations are micronuclei, anaphase bridges, dicentric chromosomes, centric rings, acentric chromosomes, chromosomal translocations, isochromosome, chromosome insertions and deletions, and wherein the telomere aberrations are selected from: the loss of one telomere, the loss of two telomeres from the same arm and the formation of telomere splits.

3. The method according to claim 1 or 2, said method comprising:
- quantifying the micronuclei and anaphase bridges using a 10x or even 40x magnification objective, and
- automatically capturing the metaphase chromosomes to detect chromosomal aberrations and/or telomere aberrations in each chromosome.

4. The method according to any one of claims 1 to 3, **characterized in that** said biological sample is a whole blood sample, marrow, or tissue cell sample.

5. The method according to any one of claims 1 to 4, wherein simultaneously labelling the telomeres and centromeres comprises:
- a single step of treating a cytogenetic slide for 1 to 3 minutes, in particular 2 minutes with a 3- 5% formaldehyde solution, in particular a 4% solution.

6. The method according to claim 5, wherein simultaneously labelling the telomeres and centromeres further comprises, after the formaldehyde treatment,
- a single step of treating the cytogenetic slide with pepsin for 3-5 minutes, in particular 4 minutes, by immersing a cytogenetic slide previously treated with formaldehyde in a pepsin solution at a concentration of 0.1-0.2ug/mL.

7. The method according to claim 6, wherein simultaneously labelling the telomeres and centromeres further comprises, after the pepsin treatment step, the following steps of:
- dehydrating the cytogenetic slide successively for 1 minute with a 50% aqueous ethanol solution, a 70% aqueous ethanol solution and pure ethanol,
- denaturing the chromosomal DNA present on the above said cytogenetic slide obtained at the end of the previous step and denaturing the peptide nucleic acid probes for telomeres and centromeres,
- hybridising for 10-30 minutes, in particular 20 minutes, at room temperature, between the denatured chromosomal DNA and the denatured peptide nucleic acid probes obtained in the previous step,
- successively washing said cytogenetic slide after hybridising.

8. The method according to any one of the preceding claims, **characterised in that** a first peptide nucleic acid probe labels the telomeres and a second peptide nucleic acid probe simultaneously labels the centromeres, the first probe and the second probe emitting a distinctive fluorescence respectively.

9. The method according to any one of the preceding claims for detecting chromosomal aberrations, said method comprising:
- labelling the telomeres and centromeres of metaphase cells with a ready-to-use solution of the nucleic acid probes,
- automatically capturing the fluorescent signals of the telomere and centromere labelling,
- analysing the image obtained in the previous step to obtain the data set: counting the number of metaphase centromeres, identifying each chromosome from its size and the ratio between the short arm (p) and the long arm (q), quantifying the signal of each telomere of each metaphase chromosome, for the detection of structural chromosome aberrations such as dicentric chromosomes, centric rings, acentric rings and different types of acentric chromosomes,
- possibly DAPI banding performed on the same metaphases, to complete the identification of chromosomes and detect single translocations,
- possibly M-FISH labelling on the same metaphases, for multicolour karyotyping to detect complex rearrangements.

10. The method according to any of the preceding claims for detecting micronuclei and anaphase bridges, said method comprising:
(i) labelling the telomeres and centromeres of interphase cells with a ready-to-use solution of the nucleic acid probes,
(ii) automatically capturing the fluorescent signals from the telomere and centromere labelling,
(iii) analysing the image obtained in step (ii) to obtain the data set : total number of micronuclei, number of micronuclei with only telomeric sequences, and number of micronuclei with both telomeric and centromeric sequences,
(iv)analysing the image obtained in step (ii) to obtain the data set: identification of anaphase bridges by detecting the presence of a DNA filament connecting two daughter cells, length of the bridge, detection of the presence or absence of centromeric or telomeric sequences in the bridge, counting the number of cells adhered.

11. A high-throughput detection kit for quantifying telomeres and detecting chromosomal aberrations and/or telomere aberrations according to any of claims 1 to 10, comprising:
- a ready-to-use solution of peptide nucleic acid probes for telomeres and centromeres,
- wash buffers required for hybridisation,
- standard slides for quantifying telomeres,
- standard slides for checking the fluorescence intensity of the microscope.
